Europäisches Patentamt

European Patent Office

Office européen des brevets

(11) Veröffentlichungsnummer: **0 645 399 A2**

## (12) EUROPÄISCHE PATENTANMELDUNG

(21) Anmeldenummer: **94104611.2**

(22) Anmeldetag: **23.03.94**

(51) Int. Cl.6: **C07K 14/415**, A61K 38/16

(30) Priorität: **25.03.93 DE 4309796**

(43) Veröffentlichungstag der Anmeldung:
**29.03.95 Patentblatt 95/13**

(84) Benannte Vertragsstaaten:
**AT BE CH DE DK ES FR GB GR IE IT LI LU MC NL PT SE**

(71) Anmelder: **LOMAPHARM Rudolf Lohmann GmbH KG Pharmazeutische Fabrik
Langes Feld 5
D-31860 Emmerthal (DE)**

(72) Erfinder: **Wagner, Hildebert, Prof.Dr.
Nelkenweg 3
D-83254 Breitbrunn/Chiemsee (DE)**
Erfinder: **Erfle, Volker, prof.Dr.
Kolbergerstrasse 7
D-81679 München (DE)**
Erfinder: **Gerhäuser, Clarissa, Dr.
Corneliusstrasse 36
D-80469 München (DE)**
Erfinder: **Ott, Holger
Taunusblick 12
D-61479 Schlossborn/Ts. (DE)**

(74) Vertreter: **Sandmair, Kurt, Dr. Dr. et al
Patentanwälte
Schwabe, Sandmair, Marx
Stuntzstrasse 16
D-81677 München (DE)**

(54) **Ribosome inaktivierendes Protein (RIP), Verfahren zu dessen Herstellung und das Ripenthaltende pharmazeutische Zubereitung.**

(57) Die Erfindung betrifft ein neues, Ribosomen-inaktivierendes Protein (P 1 oder Peponin), das aus Samen von Kürbisarten isoliert werden kann. Dieses Protein besitzt neben der bekannten Ribosomen-inhibierenden Aktivität eine die HIV Reverse Transkriptase hemmende Wirkung. Die Molmasse des Proteins beträgt ca. 31 kDa und das Protein weist einen IEP von 8 bis 9 auf.

EP 0 645 399 A2

Die Erfindung betrifft ein neues RIP (Ribosomen-inaktivierendes Protein), Verfahren zu dessen Herstellung sowie pharmazeutische Zubereitungen enthaltend dieses RIP. Das neue RIP besitzt antivirale und proliferationshemmende Eigenschaften.

Ribosomen-inaktivierende Proteine (RIPs) sind in der Lage, Ribosomen spezifisch zu inaktivieren und damit die Proteinsynthese zu stoppen. Bei den RIPs handelt es sich um Proteine, die wahrscheinlich ubiquitär in Pflanzen vorkommen. Weitere RIPs konnten aus Pilzen und Bakterien isoliert werden (vgl. mit ausführlicher Literaturbeschreibung WO 88/09123).

Bei den bislang genauer untersuchten RIPs handelt es sich um eine relativ inhomogene Klasse von Proteinen, mit Molmassen von etwa 17 000 Da bis etwa 30 000 Da.

Die biologische Aktivität der bislang untersuchten RIPs ist offensichtlich auf eine N-Glykosidaseaktivität zurückzuführen, die zur Abspaltung eines Adeninrestes der RNA der 28 S Untereinheit der 80 S Ribosomen führt. Dadurch wird die Bindung des Elongationsfaktors eF2 verhindert und die Proteinsynthese gestoppt (Endo, et al., 1987,1988).

Die biologische Aktivität der RIPs liegt in ihrer Fähigkeit in vitro selektiv die Virusreplikation zu unterbinden, wobei verschiedene RIPs selektiv die Replikation unterschiedlicher Viren zu hemmen vermögen. (Tomlinson et al., 1974; Ussery et al., 1977; Foa-Tomasi et al., 1982; Aron und Irvin, 1980)

In neuerer Zeit wurde gefunden, daß bestimmte RIPs offensichtlich in der Lage sind, in vitro die Replikation des die Immunschwächeerkrankung AIDS auslösenden HIV-Virus in HIV-infizierten T-Zellen zu verhindern (McGrath et al., 1989, 1990). Die Wirkungsweise der untersuchten RIPs ist offensichtlich auf die bereits eingangs erwähnte N-Glykosidaseaktivität der Substanzen zurückzuführen, wobei eine direkte Inhibierung der HIV Reverse Transkriptase nicht nachgewiesen werden konnte (vgl. WO 88/09123). Berichtet wurde ebenfalls über eine in vitro antitumorale Aktivität bestimmter RIPs (vgl. Tsao et al., 1986).

Aufgrund verschiedener Überlegungen, insbesondere im Hinblick auf möglichst geringe Nebenwirkungen, ist es wünschenswert, daß eine antiviralaktive Substanz ihre antivirale Wirkung möglichst direkt und spezifisch entfaltet. Im Falle der HIV-Infektion kann dies durch Hemmung spezifisch retroviraler Enzyme erfolgen, beispielsweise durch Hemmung der HIV Reverse Transkriptase.

Die bislang in der Behandlung von HIV-Infektionen verwendeten Arzneimittel besitzen eine ganze Reihe schwerster Nebenwirkungen. Wie bekannt, besitzt das in der HIV-Therapie am häufigsten eingesetzte Arzneimittel AZT eine Knochenmark supprimierende Aktivität, die den uneingeschränkten Einsatz dieses Arzneimittels stark begrenzen.

Die Anmelderin hat nun überraschend gefunden, daß ein aus Samen von Cucurbita pepo L. (Kürbissamen) isolierbares Protein über eine ausgezeichnete antivirale Aktivität verfügt und insbesondere in der Lage ist, die Replikation des HIV-Virus durch Inhibierung der HIV-spezifischen Reverse Transkriptase zu inhibieren. Dieses Protein verfügt ebenfalls über eine proliferationshemmende, insbesondere antihyperplastische Aktivität.

Aufgrund der gefundenen spezifischen und direkten Hemmung des für die HIV-Replikation verantwortlichen Enzyms kann das erfindungsgemäße Protein vorteilhaft in einem Verfahren zur Hemmung der HIV-Replikation und sonst in der Therapie von HIV-Infektionen verwendet werden.

Im Vergleich zu bekannten RIPs, insbesondere Trichosanthin, besitzt das erfindungsgemäße Protein den Vorteil, daß es neben einer der Wirkung von Trichosanthin vergleichbaren selektiven Wachstumshemmung HIV-infizierter Zellen in der Lage ist, ein für die HIV-Replikation essentielles Enzym, nämlich die HIV Reverse Transkriptase zu hemmen.

Gegenstand der Erfindung ist daher ein aus Pflanzenteilen von Cucurbita-Arten, insbesondere aus Cucurbita pepo, maxima und moschata (Monographie-Kürbissamen EB6) insbesondere aus den Samen, isolierbares Protein mit antiviraler und antiproliferativer Wirkung, Verfahren zur Isolierung dieses Proteins sowie pharmazeutische Formulierungen, die dieses Protein enthalten. Die pharmazeutischen Zubereitungen können zur Behandlung von Viruserkrankungen, bevorzugt von Erkrankungen mit retroviraler Ursache, insbesondere durch eine HIV-Infektion bedingte Erkrankungen sowie zur Behandlung von Tumorerkrankungen, bevorzugt proliferativer Tumoren, insbesondere hyperplastischer Pathogenese, bei Mensch und Tier verwendet werden.

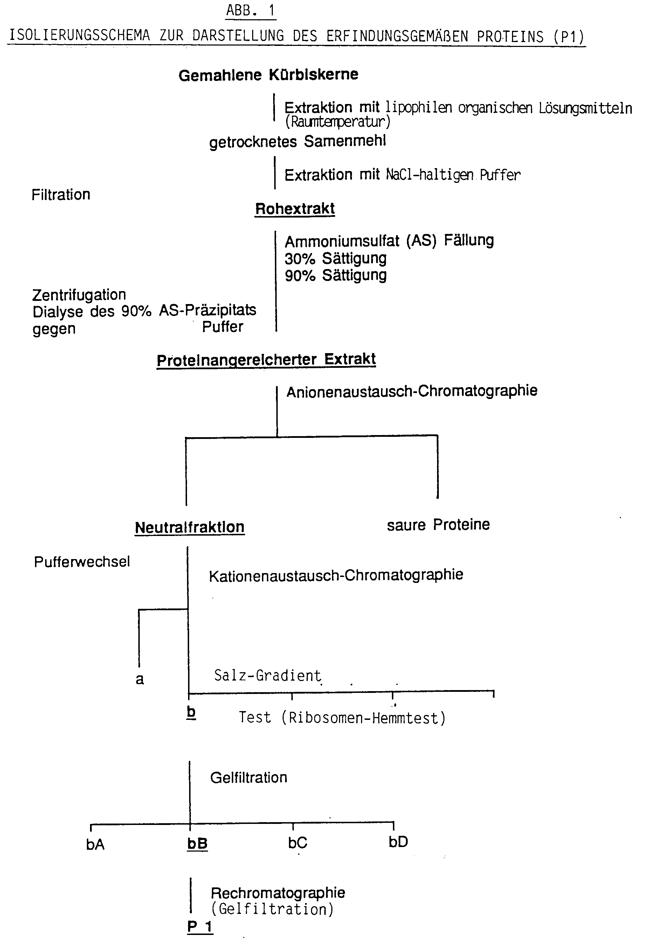

Zur Gewinnung des erfindungsgemäßen Proteins aus Kürbissamen werden diese gemahlen und zur Entfernung des Öls bei Raumtemperatur erschöpfend mit organischen Lösungsmitteln, beispielsweise Aceton, Methanol, Dichlormethan, Petroläther, n-Hexan oder Ethanol extrahiert. Das getrocknete Samenmehl wird dann mit einem NaCl-haltigen Puffer, beispielsweise PBS pH 7,2 versetzt, und über Nacht unter Rühren bei 4°C extrahiert. Nach Filtration des Ansatzes wird der gelöste Proteinanteil des Rohextrakts durch eine fraktionierende Ammoniumsulfatfällung (AS) (30% und 90% Sättigung) (vgl. Cooper, 1981) grob fraktioniert. Der nach der letzten AS-Fällung erhaltene Niederschlag wird gegen Tris-HCl Puffer dialysiert und in einer anschließenden Anionenaustauschchromatographie in saures und neutrales Material aufgetrennt. Die Neutralfraktion wird dann mittels Kationenaustauschchromatographie in neutrale und basische Fraktionen aufgetrennt und die im Hemmtest (Ribosomen-Hemmtest) aktiven Fraktionen anschließend durch Gelchromatographie weiter gereinigt. Abschließend wird das erhaltene Material nach Rechromatographie und Dialyse gegen destilliertes Wasser lyophilisiert. Eine Zusammenfassung der Isolierungsschritte zeigt die Abbildung 1.

Zur Untersuchung der biologischen Aktivität wird das aufgereinigte Protein (P 1) in einem Ribosomen-Hemmtest und einem HIV-1 RT Test eingesetzt. Im Ribosomen-Hemmtest wird die Inaktivierung von Ribosomen in einem Retikulozyten-Lysat durch das erfindungsgemäße Protein gemessen (vgl. Krawetz et al., 1983). Im HIV-1 RT Test wird die Hemmung der HIV-1 Reverse Transkriptase (HIV-1 RT) durch das erfindungsgemäße Protein P 1 bestimmt (vgl. Tan et al., 1991).

Die Ergebnisse zeigt die folgende Tabelle 1:

Tab. 1

| Ribosomen- und HIV-1 RT Hemmung durch das erfindungsgemäße Protein | | | |
|---|---|---|---|
| RIBOSOMEN-HEMMTEST | | HIV-1 RT TEST | |
| $IC_{50}$ [ng/ml] | $IC_{50}$ [nM]* | $IC_{50}$ [$\mu$g/ml] | $IC_{50}$ [$\mu$M]* |
| 5.4 ± 1.4 (n = 4) | 0.17 ± 0.04 (n = 4) | 12.7 ± 0.31 (n = 2) | 0.4 ± 0.01 (n = 2) |

\* Zur Berechnung wurde eine Molmasse von 31 kDa zugrundegelegt

Die Ergebnisse der Tabelle 1 zeigen, daß das erfindungsgemäße Protein über ausgezeichnete ribosomen-inaktivierende Wirkung und über ein sehr gutes Hemmpotential für die HIV-1 Reverse Transkriptase verfügt.

Die im Ribosomen-Hemmtest ermittelte halbmaximale Hemmkonzentration von 5,4 ng/ml liegt im Bereich der für die bekannten RIPs (z. B. Trichosanthin) in der Literatur beschriebenen Werte (Yeung et al., 1988, Stirpe et al., 1992).

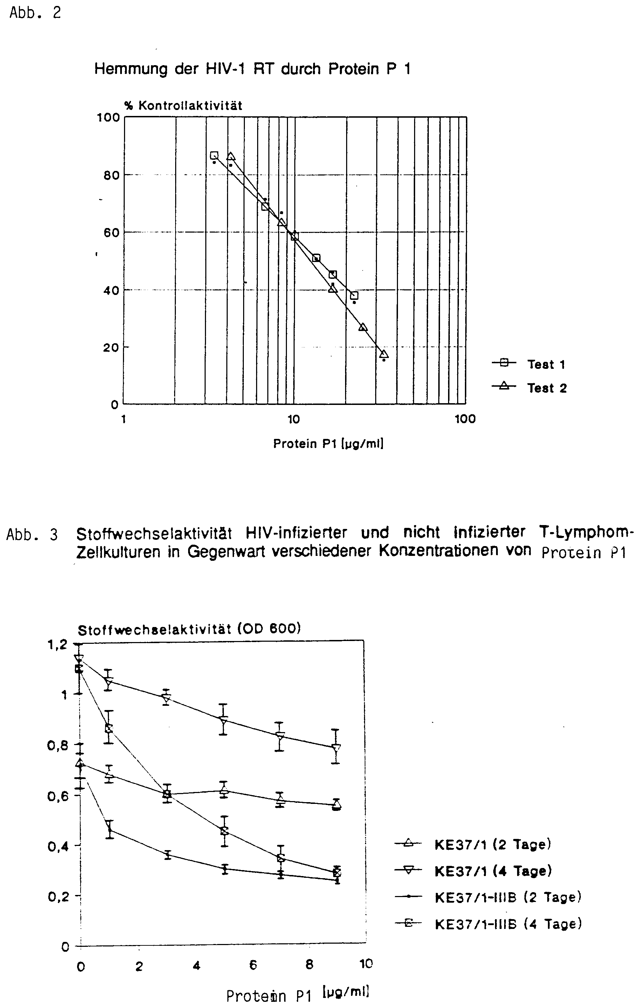

Die dosisabhängige Hemmung der HIV-1 RT durch das erfindungsgemäße Protein ist in Abb. 2 gezeigt. Der $IC_{50}$-Wert liegt eine Größenordnung unter dem $IC_{50}$-Wert von Fagaronin-Chlorid ($IC_{50}$ = 13$\mu$M), das zu den bisher aktivsten aus Pflanzen isolierten HIV-1 RT Inhibitoren zählt und als Positivkontrolle eingesetzt wurde (Tan et al., 1991).

Überraschenderweise konnte für die bekannten RIPs Trichosanthin, Bryodin und Gelonin im gleichen Testsystem bis zu einer Testkonzentration von 400 $\mu$g/ml keine Aktivität nachgewiesen werden.

Um die biologische Aktivität des erfindungsgemäßen Proteins näher zu charakterisieren, wurde das Ribosomen-Hemmpotential nach Inkubation des Proteins mit einer 0,1%igen Trifluoressigsäurelösung (1 h bei Raumtemperatur) bzw. einer 1%igen SDS-Lösung (12 h bei Raumtemperatur) untersucht. Die Ergebnisse zeigten, daß die Inkubation mit SDS eine Reduktion der Aktivität des Proteins auf ca. 10% der Ausgangsaktivität bewirkte, während die Aktivität mit Trifluoressigsäure auf 5% des Ausgangswertes reduziert wurde. Ein ähnliches Verhalten ist z. B. für Bryodin aus der Literatur bekannt (vgl. Stirpe et al., 1986).

In einem weiteren Versuchsansatz zur Untersuchung der Stabilität gegenüber Verdauungsenzymen wurde das erfindungsgemäße Protein mit Trypsin versetzt und inkubiert. Zum Vergleich wurden die bekannten RIPs Trichosanthin, Gelonin und Bryodin unter gleichen Bedingungen analysiert. Die drei untersuchten RIPs und das erfindungsgemäße Protein P 1 konnten anschließend in einer SDS-PAGE unverändert angefärbt werden, während das als Kontrolle verwendete BSA unter den angegebenen Bedingungen tryptisch gespalten wird und keine Bande im Gel detektierbar ist. Eine Stabilität von RIPs gegenüber proteolytischen Enzymen wurde bereits mehrfach in der Literatur beschrieben (vgl. Stirpe a. a. O.).

II. Physikalisch-chemische Charakterisierung

Die molekulare Masse des erfindungsgemäßen Proteins wurde mit SDS-PAGE und Gelchromatographie bestimmt. Die statistische Auswertung der elektrophoretischen Molmassenbestimmung unter denaturierenden Bedingungen in SDS-haltigen Gelen ergab einen Wert von etwa 31 ± 0,4 kDa, der mit den in der Gelchromatographie ermittelten Werten übereinstimmt.

Der isoelektrische Punkt des erfindungsgemäßen Proteins wurde mit der PEGG-Methode (Frey und Radola, 1982) in Kombination mit analytischer SDS-PAGE zu 8-9 bestimmt.

Zur Prüfung, ob das erfindungsgemäße Protein eine Glykoproteinstruktur aufweist, wurde untersucht, ob kovalent gebundene Zuckerreste nachweisbar sind. Die Untersuchung erfolgte mittels zweier spezifischer Färbemethoden, die den Nachweis von Glykoproteinen nach elektrophoretischer Trennung ermöglichen. Nach Eckhardt (Eckhardt et al., 1976) werden nach Oxidation mit Periodsäure gebildete Aldehydfunktionen mit 1-Dimethylaminonaphthalen-5-Sulfonylhydrazin zu fluoreszierenden Hydrazonen umgesetzt, die durch Reduktion mit Natriumborhydrid stabilisiert werden. Die Auswertung erfolgt unter UV-Licht bei 366 nm. Bei der Methode nach Furlan (Furlan et al., 1979) werden zum Nachweis von Glykoproteinen in Polyacrylamid oder Agarosegelen fluoreszenzmarkierte Lektine eingesetzt. In beiden Systemen konnten im erfindungsgemäßen Protein keine Zuckerreste nachgewiesen werden.

Nach einer Methode von Eckerskorn (Eckerskorn et al., 1988) wurde zur Bestimmung der Aminosäurezusammensetzung 100 pmol erfindungsgemäßes Protein mit 7,2 mol/l HCl, die 10% (V/V) Trifluoressigsäure enthielt, 3 h bei 145°C hydrolysiert. Die Aminosäuren wurden mit o-Phthalaldehyd derivatisiert und die fluoreszierenden Reaktionsprodukte HPLC-analytisch getrennt. Unter den angegebenen Bedingungen kann Prolin nicht nachgewiesen werden. Durch Vergleich der Peakflächen mit den Resultaten der Aminosäurestandardmischung konnte die Aminosäurezusammensetzung in Mol-% berechnet werden und ist in der folgenden Tabelle 2 gezeigt.

Tab. 2

| Aminosäurezusammensetzung des erfindungsgemäßen Proteins P 1 (Mol %) | | |
|---|---|---|
| | | PROTEIN P 1 |
| **D** | Asn/Asp | 9.9 |
| **E** | Gln/Glu | 9.5 |
| **S** | Ser | 8.5 |
| **G** | Gly | 7.7 |
| **T** | Thr | 8.4 |
| **H** | His | 1.1 |
| **A** | Ala | 10.1 |
| **R** | Arg | 3.3 |
| **Y** | Tyr | 5.1 |
| **V** | Val | 8.3 |
| **M** | Met | 1.0 |
| **I** | Ile | 6.0 |
| **F** | Phe | 5.1 |
| **L** | Leu | 10.1 |
| **K** | Lys | 6.0 |
| W | Trp | n.b. |
| C | Cys | n.b. |
| P | Pro | n.b. |
| n.b. nicht bestimmt | | |

N-terminale Aminosäuresequenz

Die 23 N-terminalen Aminosäuren des erfindungsgemäßen Proteins wurden durch den Edman-Abbau im Gasphasen-Proteinsequenator bestimmt und sind in der folgenden Tabelle 3 gezeigt.

## Tab. 3: N-terminale Aminosäuresequenz des erfindungsgemäßen Proteins P 1

```
              1                                                              10
P 1           Asp-    Ile-  Ala-  Leu-  Asp-  Leu-  Ser-  Thr-  Ala-  Thr-

              11                                                             20
              (Lys)-  Ala-  Ser-  Tyr-  Ser-  Ala-  Phe-  Ile-  Thr-  His

              21             23
              (Trp)-  Arg-   Asn
```

Untersuchung der Wirkung des erfindungsgemäßen Proteins auf HIV-infizierte Zellkulturen

T-Lymphozyten und Monozyten/Makrophagen sind die bevorzugten Wirtszellen für das Human Immuno Deficiency Virus im menschlichen Organismus. Um dieses Virusreservoir in HIV-infizierten Patienten zu bekämpfen, gibt es prinzipiell zwei Wege, nämlich:

a) die direkte Hemmung der Virusproduktion in den Zellen und Freisetzung aus den infizierten Zellen und

b) die selektive Hemmung des Wachstums HIV-infizierter Zellen.

Die HIV-Replikation und Möglichkeiten des Eingriffs in den Vermehrungszyklus sind gut untersucht. HIV Reverse Transkriptase Hemmstoffe, wie das Azidothymidin, hemmen selektiv die virale Neuinfektion, da die RT-Aktivität in frühen Stadien der retroviralen Infektion eine Rolle spielt. Auf bereits infizierte Zellen haben RT-Inhibitoren jedoch nur einen geringen Effekt.

Im folgenden wurde die Möglichkeit einer selektiven Beeinflussung des Wachstums chronisch HIV-infizierter T-Lymphozyten im Vergleich zu nicht infizierten T-Lymphozyten durch das erfindungsgemäße Protein untersucht.

Zur Messung der Effektivität des erfindungsgemäßen Proteins im Zellwachstumstest (MTT-Test, Mosman, 1983) werden menschlichen T-Lymphomzellen bzw. chronisch HIV-infizierten T-Lymphomzellen verschiedene Konzentrationen des Proteins P 1 als Einzeldosis am Tag 0 der Zellkultivierung in 96 well Mikrotiterplatten zugesetzt. Nach einer Inkubationszeit von 2 und 4 Tagen wird das Zellwachstum bzw. die Stoffwechselaktivität der Kulturen über die Umsetzung des Farbstoffs MTT durch Bestimmung der optischen Dichte OD 600 gemessen. Die Ergebnisse sind in der Abb. 3 dargestellt.

Wie aus der Abbildung ersichtlich ist, ist in den infizierten Kulturen ein deutlicher dosisabhängiger Einfluß des Proteins P 1 auf das Zellwachstum feststellbar. Während in der nicht infizierten Zellkultur bei einer P 1-Konzentration von 9 $\mu$g/ml eine Reduktion der Zellzahl um 25% (2 Tage) bzw. 32% (4 Tage) im Vergleich zu einer Kontrolle ohne P 1-Zusatz zu beobachten war, erreichten die HIV-1-infizierten Zellen innerhalb von 4 Tagen nicht die logarithmische Wachstumsphase.

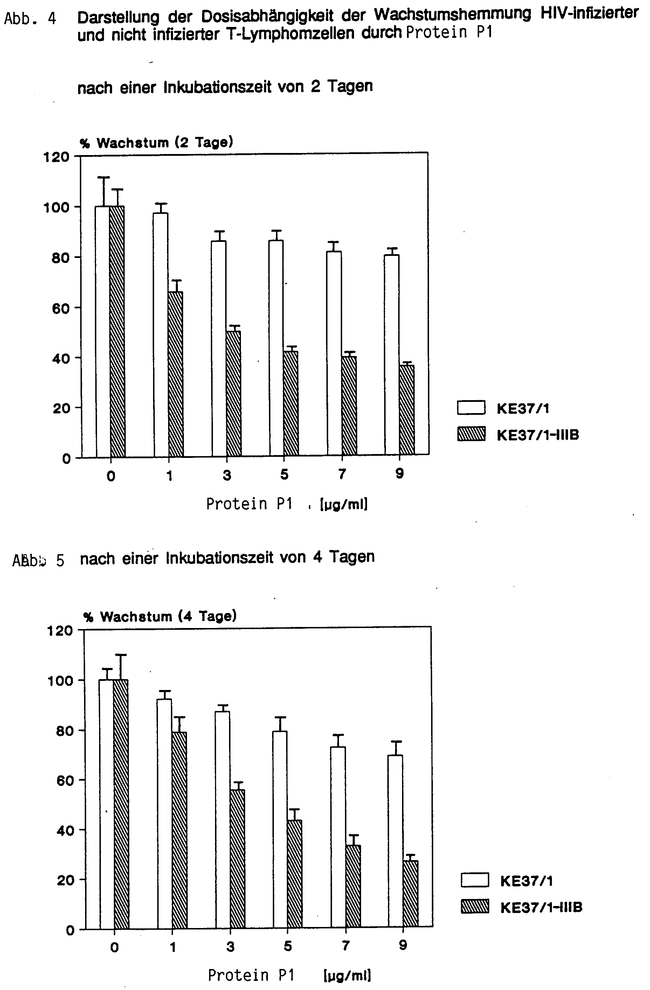

Der Einfluß verschiedener Konzentrationen von P 1 auf das Wachstum HIV-infizierter und nicht infizierter T-Lymphomzellen wurde verglichen. Die Mittelwerte der OD 600 wurden in Abhängigkeit von der OD 600 der entsprechenden unbehandelten Kulturen prozentual dargestellt, Abbildungen 4 und 5.

Der Vergleich der Ergebnisse zeigt, daß das erfindungsgemäße Protein P 1 eine selektive Hemmung des Wachstums der HIV-infizierten T-Lymphozyten bewirkt. Aus den Ergebnissen von 2 Experimenten wurde die $TCID_{50}$ (tissue culture inhibiting dose, d. h. die Konzentration, die 50% Wachstumshemmung bewirkt) in den HIV-infizierten Kulturen zu 3,0 $\mu$g/ml (2 Tage) bzw. 3,5 $\mu$g/ml (4 Tage) bestimmt. In dieser Konzentration wurde das Wachstum der nicht infizierten Zellen im Vergleich zu nicht behandelten Kulturen nur um ca. 15% reduziert. Der $TCID_{50}$-Wert in den nicht infizierten Kulturen wurde durch die Berechnung der logarithmischen Regression zu 26,9 $\mu$g/ml (4 Tage) ermittelt. Daraus läßt sich ein Selektivitätsindex SI von 7,6 ermitteln.

Nach den aufgefundenen Ergebnissen ist das erfindungsgemäße Protein P 1 in der Lage, selektiv die Proliferation chronisch HIV-infizierter T-Lymphozyten zu hemmen. Eine selektive Wachstumshemmung hat eine entscheidende Bedeutung in der Kontrolle der Verbreitung der HIV-Infektion. Infizierte T-Zellen, die HIV-Antigene an der Zelloberfläche exprimieren, sind in der Lage, nicht infizierte T-Zellen über den CD4-Rezeptor zu binden und Riesenzellen zu bilden. Die Synzytienbildung ist einer der Mechanismen, mit denen das HIV T-Zellen zerstört. Durch eine selektive Abtötung infizierter Zellen kann auch die selektive Abtötung nicht infizierter Immunzellen unterbunden werden. Dies hat insgesamt eine positive Wirkung auf die Immunabwehr HIV-infizierter Patienten.

Aufgrund der hier beschriebenen proliferations-hemmenden Wirkung des erfindungsgemäßen Proteins verfügt dieses auch über eine antihyperplastische, insbesondere antiprostatische Wirkung und stellt damit eine definierte Wirksubstanz der bislang zur Behandlung der Prostatahyperplasie eingesetzten Kürbissamen enthaltenden Präparate dar.

Das erfindungsgemäße Protein kann neben dem hier beschriebenen Isolierungsverfahren auch auf biotechnologischem Wege, insbesondere durch Zellkultur, oder auf gentechnologischem Weg durch Klonierung des entsprechenden Gens, nach den dem Fachmann bekannnten Verfahren hergestellt und isoliert werden.

Zur Isolierung des erfindungsgemäßen Proteins können selbstverständlich neben dem hier beschriebenen Verfahren auch andere dem Fachmann bekannte Verfahren zur Isolierung von Proteinen, z. B. präparative Elektrophorese, Zentrifugation, präparative Affinitätschromatographie mit entsprechenden Liganden, verwendet werden.

Obwohl es möglich ist, das erfindungsgemäße Protein allein zu verabreichen, ist es bevorzugt, es als pharmazeutische Formulierung zu verabreichen. Die Formulierungen der vorliegenden Erfindung enthalten zumindest das erfindungsgemäße Protein wie oben definiert, zusammen mit einem oder mehreren verträglichen Trägern oder Verdünnungsmitteln dafür und gegebenenfalls andere therapeutische Mittel. Jeder Träger muß "verträglich" in dem Sinne sein, daß er mit den anderen Bestandteilen der Formulierung kompatibel und für den Patienten nicht schädlich ist. Die Formulierungen schließen solche ein, die für die orale, rektale, nasale, topische (einschließend buccale und sublinguale), vaginale oder parenterale (einschließlich subkutane, intramuskuläre, intravenöse und intradermale) Verabreichung geeignet sind. Die Formulierungen können geeigneterweise in Einheitsdosis-Form dargeboten werden und nach irgendeinem dem Fachmann bekannten Verfahren hergestellt sein. Derartige Verfahren schließen die Stufe des Vereinigens des aktiven Bestandteils mit dem Träger ein, der sich aus einem oder mehreren Begleitbestandteilen zusammensetzt. Ganz allgemein werden die Formulierungen durch gleichmäßiges und inniges Inkontaktbringen der erfindungsgemäßen Verbindung mit flüssigen Trägern oder feinzerteilten festen Trägern, oder mit beiden, und anschließend, falls erforderlich, unter Formen des Produkts, hergestellt.

Beispiele:

Droge:

Zur Extraktion wurde Semen Cucurbitae incortic. tot., Semen Cucurbitae excortic. tot. (Fa. Caelo, Hilden) und Granufink Kürbissamen der Zuchtsorte Cucurbita pepo L. convar. citrullina var. styriaca (Fa. Fink, Herrenberg), verwendet.

Physikalisch-chemische Untersuchungen

N-terminale Aminosäuresequenz-Analyse

Gerät: Gasphasen-Proteinsequenator 477 A, Fa. Applied Biosystems, Foster City, CA, USA, mit isokratischer on line HPLC Trennung der Aminosäuren und computergestützter Auswertung der PTH-HPLC Analysen.
Säule: 1,6 mm I.D., Material: Superspher C 8, 4 $\mu$m, MZ-Analysentechnik, Mainz
Empfindlichkeit: Nachweis von derivatisierten Aminosäuren im Femtomol-Maßstab.
Verfahren: Beim Gasphasen-Proteinsequenator werden die für den Edman Abbau (basenkatalysierte Kupplung von Phenylisothiocyanat an die N-terminale Aminosäure eines Proteins und anschließende Hydrolyse) benötigten Reagenzien gasförmig in eine Glasreaktionskammer geleitet, die die Proteinprobe auf einem Glasfaser-Filter enthält. Die Glasreaktionskammer befindet sich in einer thermostatisierten Halterung. Am Ende eines Abbauzyklus wird die Anilinothiozolinon (ATZ)-Aminosäure extrahiert und in eine zweite Reaktionskammer überführt, in der unter Säurekatalyse die Konvertierung in ein stabiles Phenylthiohydantoin (PTH)-Derivat stattfindet.

Chromatographische Verfahren

Normaldruck-Säulenchromatographie

Alle chromatographischen Verfahren werden bei 5 - 7°C durchgeführt. Allen Pufferlösungen, mit Ausnahme des flüchtigen Ammoniumacetat-Puffers, wurde nach Einstellung des pH-Wertes 0,02% Natriumazid als Konservierungsmittel zugesetzt.

Die Proben werden vor der Applikation zentrifugiert (10 000 g, 5 min, 4°C) und mit Hilfe der Pumpe über einen dünnen Teflonschlauch direkt auf die Säulenoberfläche aufgebracht.

Apparative Daten:

Detektoren: Uvicord S II LKB 2238, UV-Filter 280 nm, Fa. LKB, Schweden; Altex Rodel 151 Dual Wavelength UV Detector mit Altex Dual Wavelength Optical Unit
Schreiber: Flachschreiber BD 41, Fa. Kipp und Zonen, Solingen
Pumpe: LKB 2232 Mikroperpex S Pumpe, Fa. LKB, Bromma, Schweden
Gradientenmischer: GM-1(2 x 250 ml), Fa. Pharmacia, Freiburg
Fraktionssammler: LKB 2070 Ultrorac II, Fa. LKB, Bromma, Schweden
Fraktionen: à 20 min

Gelmaterialien:

DEAE 23 SS-Cellulose Servacel, Fa. Serva, Heidelberg; CM 23-Cellulose Servacel, Fa. Serva, Heidelberg; CM-Sepharose CL-6B, Fa. Pharmacia AB, Freiburg; Sephadex G 75 Superfein (Trennberich 3 bis 70 kDa), Fa. Pharmacia, Freiburg; Blue Sepharose CL-6B, Fa. Pharmacia AB, Freiburg; (Sephadex G 50 Superfein (Trennbereich 1,5 bis 30 kDa), Fa. Pharmacia, Freiburg), (S-Sepharose Fast Flow, Fa. Pharmacia, Freiburg)

Eluentien:

10 mM Tris-HCl Puffer pH 7,6; 1 M NaCl in 10 mM Tris-HCl Puffer pH 7,6; 10 mM $NaH_2PO_4$ Puffer pH 6,0; linearer NaCl-Gradient in 10 mM $NaH_2PO_4$ Puffer pH 6,0; PBS pH 7,2 (phosphatgepufferte Kochsalzlösung aus 150 mM NaCl in 10 mM $NaH_2PO_4$ Puffer pH 7,2); 50 mM Ammoniumacetat Puffer pH 6,75
Säulen: Chromatographiesäulen XK 16 und XK 24 mit passenden Adaptern AK16 und AK24, Fa. Pharmacia, Freiburg

| | |
|---|---|
| A = 3,2 cm$^2$ | l = 20 cm |
| A = 5,3 cm$^2$ | l = 25 cm |
| A = 4,5 cm$^2$ | l = 11,5 cm |
| A = 7,1 cm$^2$ | l = 18 cm |
| A = 4,5 cm$^2$ | l = 54 cm |
| A = 2,0 cm$^2$ | l = 8,5 cm |
| (A = 2,0 cm$^2$ | l = 85 cm) |

Aminosäureanalyse

Gerät: Aminosäureanalysator Beckmann 6300
Die apparativen Daten zur qualitativen und quantitativen OPA-Aminosäure-Bestimmung finden sich in der zitierten Literatur (Eckerskorn et al., 1988)

Elektrophoretische Arbeitsmethoden

Analytische SDS-Polyacrylamid Gel Elektrophorese (SDS-PAGE)

Apparative Daten:

Stromversorger: LKB 2197 Constant Power Supply, Fa. LKB, Schweden
Kammern: LKB 2117 Multiphor I, Fa. LKB, Bromma, Schweden; LKB 2117-001 Multiphor II, Fa. LKB, Bromma, Schweden; Biometra Minigel G 41 (Vertikal-Elektrophorese), Fa. Biometra, Göttingen

Gel-Stammlösungen:

| | | |
|---|---|---|
| a. Acrylamid/Bis: (30% T/3% C) | Acrylamid<br>Bisacrylamid<br>$H_2O$ dest. ad | 29,1 g<br>0,9 g<br>100 ml |
| | (kühl lagern, 4 Wochen haltbar) | |
| b. Gelpuffer pH 8,8: (4fach konz.) | Tris (1,5 M)<br>SDS<br>$H_2O$ dest. ad<br>pH 8,8 mit 4 N HCl $NaN_3$ | 18,18 g<br>0,40 g<br>100 ml<br>0,01 g |
| c. Gelpuffer pH 6,8: (4fach konz.) | Tris (0,5 M)<br>SDS<br>$H_2O$ dest. ad<br>pH 6,8 mit 4 N HCl $NaN_3$ | 6,06 g<br>0,40 g<br>100 ml<br>0,01 g |
| d. TEMED 10% | TEMED<br>$H_2O$ dest. ad | 100 $\mu$l<br>1 ml |
| e. APS 4% | Ammoniumpersulfat<br>$H_2O$ dest. ad | 40 mg<br>1 ml |

Die Lösungen d und e sind 1 Woche bei 4 °C haltbar

Elektrodenpuffer:

| | | |
|---|---|---|
| Kathodenpuffer: Tris/Glycin/SDS (10fach konz.) | Tris (250 mM)<br>Glycin<br>SDS<br>$NaN_3$<br>$H_2O$ dest. ad | 30,28 g<br>144,00 g<br>10,00 g<br>1,00 g<br>1000 ml |
| Anodenpuffer: Tris-HCl pH 8,4 (10fach konz.) | Tris (250 mM)<br>$H_2O$ dest. ad<br>pH 8,4 mit 4 N HCl $NaN_3$ | 30,28 g<br>1000 ml |

Probenpuffer:

| a. denaturierend | Gelpuffer pH 8,8 | 2,5 ml |
| | SDS | 1,0 g |
| | Glycerin | 50,0 ml |
| | Bromphenolblau | 10,0 mg |
| | $H_2O$ dest. ad | 100 ml |
| b. reduzierend (GERS-BARLAG 1989)) | 0,5 M Tris-HCl Puffer | 0,3 ml |
| | SDS Lösung 10% | 2,0 ml |
| | 2-Mercaptoethanol | 1,0 ml |
| | 10 M Harnstoff | 6,0 ml |
| | Glycerin | 1,0 ml |
| | Bromphenolblau | 2,0 mg |

Gelstabilisierung: Polymerisation auf die hydrophile Seite eines Gel-Bond PAG Film, Fa. Biozym, Hameln

| Markerproteine: | Rinderserumalbumin | 67 kDa | 0,1 mg |
| | Carboanhydrase | 29 kDa | 0,2 mg |
| | Myoglobin Pferd | 17,8 kDa | 0,1 mg |
| | Probenpuffer a ad | | 1,0 ml |

Horizontale SDS-PAGE mit Porengradient

Zunächst wird eine Gelgießkammer aus zwei Glasplatten, der Stabilisierungsfolie und einem Spacer (Abstandhalter aus 4 (0,048 cm) bzw. 5 (0,06 cm) Lagen Para-Film) zusammengesetzt und an drei Seiten mit Klammern fixiert. Der Gießstand wird mit Hilfe einer Wasserwaage ausgerichtet, um eine Verzerrung des Porengradienten zu vermeiden.

Das Gesamtgelvolumen wird bestimmt, indem der Gießstand mit $H_2O$ dest. gefüllt und das Volumen anschließend gemessen wird. In analoger Weise wird das Volumen einer 3 cm hohen Plateauschicht ermittelt.

Die Gele werden mit Hilfe eines Gradientenmischers aus zwei Gellösungen mit definierten Acrylamid-Konzentrationen (T) gegossen. Die Lösungen werden aus den Stammlösungen a - d nach folgender Rezeptur gemischt:

| | 'schwere Lösung' (10% T / 3% C) | 'leichte Lösung' (22,5% T / 3% C) |
| --- | --- | --- |
| Acrylamid/Bis | 4,0 ml | 7,5 ml |
| Glycerin (87%) | 3,6 ml | |
| Gelpuffer pH 8,8 | 3,0 ml | 2,5 ml |
| TEMED 10% | 60 $\mu$l | 50 $\mu$l |
| $H_2O$ dest. ad | 12 ml | 10 ml |
| Einsatz | 3,0 ml (Plateau) + 6,5 ml (Gradient) | 6,5 ml (Gradient) |
| APS 4% | 80 $\mu$l | 50 $\mu$l |

Die Gel-Lösungen werden in der angegebenen Reihenfolge zusammenpipettiert und vorsichtig gemischt (Vermeidung der Schaumbildung durch SDS). Die Dichte der 'schweren' Lösung (geringere Acrylamid-Konzentration, größere Poren) wird durch den Zusatz von Glycerin erhöht, um die Bildung des Porengradienten zu gewährleisten. Die angegebenen Volumina werden in den Gradientenmischer gegeben (schwere Lösung am Auslaß, Verbindungskanal entlüftet). Die 'schwere' Lösung wird ständig mit einem kleinen Magnetrührer gerührt. Durch die Zugabe der entsprechenden Ammoniumpersulfat-Mengen wird die Polymerisation gestartet.

Zunächst läßt man das der Plateauschicht entsprechende Volumen der 'schweren' Lösung in den Gießstand fließen. Anschließend wird durch Öffnung des Verbindungskanals der Gradient gestartet und der Gießvorgang langsam fortgesetzt, bis der Gießstand ganz gefüllt ist (Dauer ca. 3 min).

Zur Ausbildung des Gradienten muß das Gel ca. 15 min bei Raumtemperatur stehen. Die Gelpolymerisierung kann bei Raumtemperatur über Nacht oder innerhalb von 30 min im Trockenschrank bei 50°C vorgenommen werden. Nach abgeschlossener Polymerisation wird der Gießstand auseinandergenommen und das Gel auf die mit Kerosin imprägnierte Kühlplatte der Elektrophoresekammer luftblasenfrei aufgelegt. Das Plateau wird dabei an der Kathodenseite angelegt. Mit Hilfe von Silikon-Applikationsstreifen werden die Proben im Bereich der Plateauschicht ca. 1,5 cm vom Rand entfernt aufgebracht.

Gel-Dimension:    24,2 x 11 x 0,048 cm,
                  Trenndistanz: 8 cm

Proben: Volumen: 8 - 10 $\mu$l, Konzentration 1-2 mg/ml Probenpuffer a oder b, Denaturierung durch Inkubation im kochenden Wasserbad (5 min in Eppendorfgefäßen)


Elektrophoresebedingungen:


Spannung: 1200 V max.

Strom: 50 mA max.

Leistung: 40 Watt max.

Kühlung: Glasplatte (Kerosin als Kühlvermittler), fließendes Leitungswasser

Dauer: Die Elektrophorese wird abgebrochen, sobald die Bromphenolblau-Bande an der Anodenseite aus dem Gel wandert (ca. 120 min)

SDS-PAGE unter diskontinuierlichen Bedingungen nach Laemmli, 1970 (modifiziert nach Fairbanks et al., 1971)

Das Gel setzt sich aus einem ca. 1,5 cm langen Sammelgel und einem 5,5 cm langen Trenngel zusammen. Das großporige Sammelgel erleichtert den Probeneintritt hochmolekularer Proteine in das Gel. Beim Übergang vom Sammelgel in das Trenngel werden die Proteine durch den Porengrößen- und pH-Unterschied aufkonzentriert und man erhält schärfere Banden.

Die Gele werden in einem aus zwei Glasscheiben bestehenden Gießstand gegossen, der im Anschluß direkt in die Elektrophoresekammer eingebaut wird. Zur Erhöhung der mechanischen Stabilität wird ebenfalls eine Gel-Bond PAG Folie verwendet.

Die Gellösungen werden nach der folgenden Rezeptur bereitet:

|  | Trenngel 12% T | Sammelgel 3,75% T |
|---|---|---|
| Acrylamid/Bis | 2,00 ml | 0,5 ml |
| Glycerin (87%) | 1,50 ml | 1,0 ml |
| Gelpuffer a pH 8,8 | 1,25 ml | - - |
| Gelpuffer b pH 6,8 | - - | 1,0 ml |
| H$_2$O dest | 0,25 ml | 1,5 ml |
| TEMED 10% | 30 $\mu$l | 25 $\mu$l |
| APS 4% | 40 $\mu$l | 45 $\mu$l |

Zunächst werden die Komponenten der Trenngellösung vorsichtig gemischt und die Polymerisation durch Zugabe von Ammoniumpersulfat gestartet. Die Lösung wird in die Kammer gefüllt und mit Isopropanol überschichtet. Nach Beendigung der Polymerisationszeit (12 h bei Raumtemperatur) wird das Isopropanol mit Hilfe einer Spritze abgezogen und die Geloberfläche mit einem Waschpuffer, bestehend aus 15 ml Glycerin, 12,5 ml Trenngelpuffer pH 8,8 und 22,5 ml H$_2$O dest., 3 - 4 mal gründlich gewaschen. Der Waschpuffer wird abgegossen, letzte Pufferreste werden mit einem Filterpapier abgesaugt.

Die Polymerisation der inzwischen vorbereiteten Sammelgellösung wird durch APS-Zugabe gestartet. Die Lösung wird auf das Trenngel pipettiert. An der Oberkante der Gießkammer wird zur Erzeugung von Probentaschen ein Teflon-Kamm luftblasenfrei in das noch flüssige Sammelgel gedrückt. Nach einer Polymerisationszeit von 45 min wird der Kamm entfernt und die Taschen mit 1:4 verdünntem Sammelgelpuffer pH 6,8 mehrere Male gewaschen. Die Probenapplikation erfolgt mit Hilfe einer Hamiltonspritze (25 $\mu$l). Die Silikondichtung des Gießstandes wird entfernt. Das Gel wird mit den Glasplatten vorschriftsgemäß in die Elektrophoresekammer eingebaut. Beide Puffertanks sollten bereits zu ca. 2/3 mit dem 1:10 verdünnten Kathodenpuffer gefüllt sein. Beim Einbringen der Glasplatten in den unteren Tank ist darauf zu achten, daß sich keine Luftblasen im Spalt zwischen den Platten befinden. Die Platten werden mit Klammern befestigt, die Puffertanks bis ca. 0,5 cm unter den oberen Rand aufgefüllt und die Abdeckplatte angebracht.

Gel-Dimension:      8 x 7 x 0,048 cm
Trenndistanz: 5,5 cm

Proben:           Volumen: 10-20 $\mu$l, Konzentration ca. 1 mg/ml Probenpuffer a, Denaturierung durch Inkubation im kochenden Wasserbad (5 min in Eppendorfgefäßen)

Elektrophoresebedingungen:

Spannung: max
Strom: 10 mA konstant
Leistung: max.
Kühlung: Luft
Dauer: Die Elektrophorese wird abgebrochen, sobald die Bromphenolblau-Bande an der Anodenseite aus dem Gel wandert (ca. 180 min)
Präparative isoelektrische Fokussierung (IEF) (nach Frey und Radola, 1982)
Apparative Daten: LKB 2117-001 Multiphor II Kammer, Fa. LKB, Schweden
Elektrodenpuffer: Anoden Lösung, Fa. Serva, Heidelberg; Kathoden Lösung, Fa. Serva, Heidelberg
Gel: Sephadex G 200 Superfein, versetzt mit 2% (G/V) Trägerampholinen (pI 3-10 oder pI 9-11, Fa. Serva)

Durchführung:

Sephadex G 200 Superfein wird 3 Tage lang in $H_2O$ dest. vorgequollen, anschließend mit 20 Volumen $H_2O$ dest. ionenfrei gewaschen und als gerade noch gießbare Suspension bei 4°C gelagert. Die für eine 1 mm dicke Gelschicht benötigte Gel-Menge wird aus der Plattendimension errechnet (+ ca. 20% Überschuß), mit 2% (G/V) Trägerampholinen versetzt und gut gemischt. Das Gel wird kurz am Wasserstrahlvakuum entgast und anschließend gleichmäßig dünn auf einer dünnen, mit Aceton gereinigten Glasplatte (260 x 60 x 1 mm) verteilt. Zur Verfestigung der Gelschicht wird kurzzeitig mit dem Föhn getrocknet, bis die Schicht bei einer Neigung von 45° stabil ist.

Gel-Dimension:      26 x 6 x 0,1 cm
Trenndistanz: 20 cm

Probe: ca. 80 mg in 800 $\mu$l $H_2O$ dest., Probenapplikation an der Anodenseite
Elektrodenpuffer: wie vorhin beschrieben

Fokussierungsbedingungen:

Prefokussierung:

Spannung: 400 V konstant (20 V/cm)
Strom: max.
Leistung: max.
Dauer: 16 h

Elektrofokussierung:

Spannung: 2000 V konstant (100 V/cm)
Strom: max.
Leistung: max.
Dauer: 2 - 3 h
Kühlung: Aluminiumplatte, fließendes Leitungswasser
Nach Abschluß der Fokussierung wird das Gel in 20 Streifen à 1 cm geteilt, das Gelmaterial in Eppendorfgefäße überführt und mit je 500 $\mu$l $H_2O$ dest. versetzt. Die Proben werden 2 h geschüttelt, anschließend wird der pH-Wert nach kurzer Zentrifugation (10 000 rpm, 5 min) in den Probenüberständen gemessen. Zur Detektion fokussierter Proteine werden 10 $\mu$l Aliquots entnommen, mit 10 $\mu$l Probenpuffer b versetzt und SDS-elektrophoretisch untersucht. Der Proteingehalt der Proben wird nach Smith (1985) bestimmt.

Färbemethoden

| Proteinfärbung mit Coomassie Brilliant Blue | | |
|---|---|---|
| | | Dauer |
| a. Fixierlösung: | Trichloressigsäure 20 g<br>$H_2O$ dest. ad 100 ml | 30 min |
| b. Waschlösung:<br>c. Färbelösung:<br><br>d. Entfärbelösung:<br>e. Imprägnierlösung: | Methanol-Eisessig-Wasser (40 + 10 + 50)<br>Methanol-Eisessig-Wasser (40 + 10 + 50) mit<br>0,1% Coomassie Brilliant Blue R 250<br>Methanol-Eisessig-Wasser (25 + 10 + 65)<br>Methanol-Wasser-Glycerin (70 + 26 + 4) | 3 x 20 min<br>12 h<br><br>3 x 2 h<br>1 min |

Einsatz pro Arbeitsschritt ca. 250 ml (24 x 11 cm Gel) bzw. 100 ml (Minigel)

Zur Aufbewahrung werden die Gele mit feuchter Cellophanfolie luftblasenfrei bedeckt, an den Längskanten beschwert und bei Raumtemperatur getrocknet (ca. 12 h). Die Cellophanfolie verhindert ein Reißen des Gels während des Trocknungsvorgangs und ermöglicht anschließend das Ablösen der Stabilisierungsfolie.

Spezielle Färbemethoden zum Nachweis von Glykoproteinen

A. Anfärbung der SDS-PAGE mit Dansylhydrazin (Eckhardt et al., 1976)

| | | Einsatz, Dauer |
|---|---|---|
| a. Fixierung: | Trichloressigsäure 20 g<br>$H_2O$ dest. ad 100 ml | 250 ml, 30 min |
| b. Spül-Schritt:<br>c. Oxidationsschritt:<br><br>d. Wasch-Schritt:<br>e. Reduktion:<br><br>f. Wasch-Schritt:<br>g. Färbeschritt:<br>Wasserbad 60°C langsam<br>schütteln, Ansatz verschließen<br>h. Reduktion:<br>i. Wasch-Schritt:<br>k. Entfärbung:<br>l. Imprägnierung + Trocknen | Ethanol-Eisessig-Wasser (40 + 5 + 55)<br>0,7% (G/V) Perjodsäure in<br>Eisessig-Wasser (5 + 95)<br>dest. Wasser (500 ml)<br>0,5% (G/V Natriummetabisulfit in<br>Eisessig-Wasser (5 + 95)<br>vgl. d.<br>(1) HCl-DMSO (0,06 + 1000)<br>(2) Dansylhydrazin/DMSO (2 mg/ml)<br>je 125 ml (1) und (2) mischen<br>Natriumborhydrid/DMSO (0,2 mg/ml)<br>vgl. d.<br>Eisessig-Wasser (10 + 990)<br>vgl. Coomassiefärbung | 3 x 250 ml, 12 h<br>250 ml, 2 h<br><br>3 x 10 min<br>250 ml, 2 h<br><br><br><br>250 ml, 2 h<br><br>250 ml, 30 min<br><br>250 ml, 12 h |

Die Detektion der ursprünglich glykosylierten Proteinbanden erfolgt durch Fluoreszenzanregung bei 366 nm. Zur Zuordnung der gefärbten Banden wird ein 'Parallel'-Gel mit Coomassie gefärbt.

B. Anfärbung der SDS-PAGE mit fluoreszenzmarkiertem Con A (Furlan et al., 1979)

Präparation von FITC-Con A:

Con A (5 mg/ml in 50 mM Phosphatpuffer pH 9,5) wird 20 h bei 25°C mit FITC (Fluorescein-Isothiocyanat) (Endkonzentration 100 µm/ml) inkubiert. Anschließend wird das Reaktionsprodukt mit mehreren Wechseln gegen einen Dialysepuffer dialysiert (0,1 M NaCl in 50 mM Tris-HCl Puffer pH 7,0 (ohne Azid), nach pH-Wert Einstellung Zusatz von 1 mM $CaCl_2$ und 1 mM $MnCl_2$). Das Ausmaß der Kopplung kann durch Absorptionsmessungen bei UV 280 nm und 495 nm überprüft werden. Das Verhältnis der Absorptionen sollte bei ca. 0,9 liegen. Die Konzentration an FITC-Con A im Puffer wird auf ca. 1 mg/ml eingestellt.

Durchführung der Färbung:

Die mit 20% Trichloressigsäurelösung fixierten Gele werden mit 4 x 250 ml des Dialysepuffers (s.o.) gewaschen. Anschließend werden die Gele 12 h mit der FITC-Con A Pufferlösung inkubiert und 48 h mit Dialysepuffer entfärbt.
Auswertung: s.o.

Molmassenbestimmung

SDS-PAGE

Apparative Daten, Durchführung: vgl. 'Horizontale SDS-PAGE mit Porengradient'
Markerproteine ($M_r$): Fa. Serva, Heidelberg

| Myoglobin (Pferd) | 17,8 kDa | Ovalbumin | 45 kDa |
| Chymotrypsinogen | 25 kDa | Rinderserumalbumin | 67 kDa |
| Carboanhydrase | 29 kDa | Transferrin | 80 kDa |
| Aldolase | 40 kDa | | |

Konzentration 0,05 mg/ml Probenpuffer a
Aus den lg $M_r$ und den Wanderungsstrecken der Proteine im Gel wird eine Eichgerade konstruiert.

Gelfiltrations-Chromatographie

Apparative Daten: vgl. 'Normaldruck-Säulenchromatographie'
Flußrate: 15 ml/h
Eluens: PBS pH 7,2
Gelmaterial: Sephadex G 75 Superfein (Fa. Pharmacia, Freiburg)
Säule: A = 4,5 cm² l = 54 cm

| Eichung: | Dextran Blau | 2 Mio. Da (Ausschlußvolumen $V_O$) |
| | Rinderserumalbumin | 67 kDa |
| | Ovalbumin | 45 kDa |
| | Carboanhydrase | 29 kDa |
| | Myoglobin (Pferd) | 17,8 kDa |
| | DNP-Alanin | 0,225 kDa (Totalvolumen $V_T$) |

Berechnung:

$$K_{av}\text{-Wert} = \frac{(V_E - V_0)}{(V_T - V_0)}$$

$V_E$:　　Elutionsvolumen
$V_O$:　　Ausschlußvolumen
$V_T$;　　Totalvolumen

Im linearen Trennbereich des verwendeten Gelmaterials ist der lg $M_r$ dem $K_{av}$-Wert direkt proportional. Die Berechnung der Molmasse eines Proteins erfolgt durch graphische Ermittlung des lg $M_r$ nach Bestimmung von $K_{av}$ aus dem Elutionsvolumen $V_E$ des Proteins.

Zentrifugation

Geräte: Varifuge 20 RS, Fa. Heraeus Sepatech, Osterode/Harz; Biofuge 13 (für Eppendorfgefäße), Fa. Heraeus Sepatech, Osterode/Harz

Rotoren: Festwinkelrotor HFA 12.500 ($r_{av.}$ 10,5 cm) (präparativ), Festwinkelrotor HFA 22.500 ($r_{av.}$ 7,15 cm) (Klarzentrifugation von Extrakten, max. 48 745 g), Winkelrotor 24 x 1,5 ml für Biofuge 13, Osterode/Harz

Dialyse

Dialyseschläuche: Visking Dialysierschlauch verschiedener Dimension, Fa. Serva, Heidelberg
mittlerer Porenradius: 24 Å
Ausschlußgrenze: 6 bis 10 kDa
Dauer: mindestens 36 h bei 4 °C mit 3-4 Wechseln
Die Dialyse erfolgte gegen doppelt demineralisiertes (dd) Wasser oder die angegebene Pufferlösung.
Cucurbita pepo L.

Isolierung von Peponin (Protein P 1)

Extraktion hochmolekularer Inhaltsstoffe

600 g hartschalige, geschälte Kürbissamen wurden gemahlen und bei Raumtemperatur zur Entfernung lipophiler Inhaltsstoffe mit Aceton erschöpfend extrahiert. Ausbeute an entfettetem, getrockneten Samenmehl: 50%. Alle weiteren Schritte wurden bei 4 °C ausgeführt. 300 g entfettetes Samenmehl wurden mit 2 l PBS pH 7,2 versetzt, nach einer Quellzeit von 2 h im Waring Blender bei 4 °C homogenisiert und unter Rühren 12 h extrahiert. Durch Zentrifugation (8 000 g, 20 min, 4 °C) wurden unlösliche Bestandteile abgetrennt.

Ammoniumsulfat Fällungen

Der gelblich gefärbte Überstand wurde unter ständigem Rühren portionsweise mit $(NH_4)_2SO_4$ bis zu einer Sättigung von 30% versetzt (zur Berechnung der benötigten Salzmengen vgl. Tabelle in Cooper, 1981). Nach 6 h wurde die 30% Fällung abzentrifugiert (10 000 g, 20 min, 4 °C) und verworfen. Der Überstand (1520 ml) wurde bis zu einer Sättigung von 90% mit $(NH_4)_2SO_4$ versetzt. Das Verfahren entsprach dem der 30% Fällung. Nach vollständiger Auflösung des Salzes wurde der Ansatz mindestens 12 h bei 4 °C aufbewahrt. Anschließend wurden die ausgefällten Proteine durch Zentrifugation (8 000 g, 60 min, 4 °C) gewonnen. Das Proteinpellet wurde in 10 mM Tris-HCl Puffer pH 7,6 aufgenommen und 48 h gegen denselben Puffer dialysiert (5 Wechsel) (= proteinangereicherter Extrakt). Ein während der Dialyse auftretender Niederschlag wurde durch Zentrifugation (10 000 g, 30 min, 4 °Cl) abgetrennt und verworfen.

Anionenaustausch-Chromatographie

Der proteinangereicherte Extrakt wurde auf die Säule gepumpt und die Neutralfraktion zunächst mit 10 mM Tris-HCl Puffer pH 7,6 eluiert. Die Elution einer sauren Fraktion erfolgte anschließend mit 1 M NaCl in demselben Puffer.
Gelmaterial: DEAE 23 SS-Cellulose Servacel, Fa. Serva, Heidelberg
Säule: A = 7,1 cm², l = 18 cm, äquilibriert mit 10 mM Tris-HCl Puffer pH 7,6
Eluens: 10 mM Tris-HCl Puffer pH 7,6; 1 M NaCl in 10 mM Tris-HCl Puffer pH 7,6
Flußrate: 20 ml/h
Auftragemenge: 525 ml proteinangereicherten Extrakt
Die Neutralfraktion (600 ml) wurde gegen 10 mM Natriumphosphat Puffer pH 6,0 dialysiert (36 h, 4 x 5 l). Die saure Fraktion zeigte im Ribosomen-Hemmtest in einer Konzentration von 1,0 $\mu$g/ml keine Aktivität und wurde nicht weiter untersucht.

Kationenaustausch-Chromatographie der Neutralfraktion

Mit 10 mM $NaH_2PO_4$ Puffer pH 6,0 wurde die nicht am Säulenmaterial gebundene Fraktion a eluiert. Zur Elution der basischen Fraktionen b bis e wurde ein linearer NaCl-Gradient (0 - 0,5 M) eingesetzt. Die Fraktionen wurden gegen dd Wasser dialysiert und gefriergetrocknet.

Gelmaterial: CM 23-Cellulose Servacel, Fa. Serva, Heidelberg,

Säule: A = 4,5 cm$^2$, l = 11,5 cm, äquilibriert mit 10 mM NaH$_2$PO$_4$ Puffer pH 6,0

Eluens: 10 mM NaH$_2$PO$_4$ Puffer pH 6,0

Gradient: 0 bis 0,5 M NaCl in 10 mM NaH$_2$PO$_4$ Puffer pH 6,0 (2 x 130 ml)

Flußrate: 15 ml/h

Auftragemenge: 3 x 200 ml

Ausbeute (3 Läufe): a (nicht gebunden): 1240 mg; b: 159,4 mg; c: 202,9 mg; d: 277,8 mg; e: 260 mg

Gelfiltrations-Chromatographie der Fraktion b

Gelmaterial: Sephadex G 75 Superfein (Trennbereich 3 bis 70 kDa)

Säule: A = 4,5 cm$^2$, l = 54 cm

Eluens: PBS pH 7,2 (150 mM NaCl in 10 mM NaH$_2$ PO$_4$ pH 7,2)

Flußrate: 20 ml/h

Auftragemengen: Lauf 1: 41,3 mg/1,5 ml PBS pH 7,2; Lauf 2: 41,7 mg/1,5 ml PBS pH 7,2; Zentrifugation der Probe (5000 g, 5 min)

Ausbeute (Lauf 1 + 2): bA: 19,5 mg, bB: 8,8 mg, bC: 4,0 mg, bD: 3,9 mg

Aus Fraktion bB konnte durch Rechromatographie an Sephadex G 75 SF Protein P 1 (Peponin) rein dargestellt werden. Das Eluat wurde gegen dd Wasser dialysiert und gefriergetrocknet.

Flußrate: 15 ml/h

Auftragemenge: 7 mg Fraktion bB/1,5 ml PBS pH 7,2

Ausbeute: 3,1 mg (44,24% ≙ 0,0005% der eingesetzten Drogenmenge)

Stabilitätsprüfung von Protein P 1

a. Inkubation mit 0,1% Trifluoressigsäure

Protein P 1 wird in einer Lösung von 0,1% TFA in Acetonitril-Wasser (60 + 40) gelöst (1 mg/ml) und 1 h bei Raumtemperatur inkubiert. Anschließend wird der Ansatz zur Testung auf verbleibende Ribosomen-Hemmaktivität mit 10 mM Tris-HCl Puffer pH 7,6 verdünnt. Als Kontrolle wird die TFA-Lösung 1:10 mit 10 mM Tris-HCl Puffer pH 7,6 verdünnt.

b. Inkubation mit 1%iger SDS Lösung

Protein P 1 (1 mg/ml) wird für 16 h bei Raumtemperatur mit einer 1%igen SDS Lösung in 10 mM Tris-HCl Puffer pH 7,6 inkubiert. Die Auswertung erfolgt über die Aktivitätsmessung im Ribosomen-Hemmtest im Vergleich zu einer unbehandelten Proteinprobe und der verdünnten Reagenzlösung ohne Proteinzusatz.

c. Proteinabbau mit Trypsin

Trypsin aus Rinderpankreas (Sequenzing grade, Fa. Boehringer Mannheim) wird in 1 mM HCl gelöst (1 mg/ml). Die Trypsinlösung wird in einer Konzentration von 2% (V/V) einer Lösung von Protein P 1 oder Vergleichsproteinen in 10 mM Tris-HCl Puffer pH 7,6 (1 mg/ml) zugesetzt (in verschließbaren Eppendorfgefäßen). Die Proben werden 17 h bei 37°C im Wasserbad inkubiert, anschließend kurz zentrifugiert (Biofuge 13, 6000 rpm, 2 min). Die Auswertung erfolgt SDS-elektrophoretisch.

Aminosäureanalyse des Proteins P 1

Das gelöste Protein P 1 (100 pmol P1) wurde auf Glasfaser-Filter pipettiert und unter Vakuum getrocknet. Zur GasphasenHydrolyse wurden die Glasfaser-Filter in kleinen Glasröhrchen (2,5 x 8 mm) in ein äußeres Hydrolysegefäß gestellt, das 2 ml einer Hydrolyselösung (7,2 M HCl, 10% TFA, 0,1% Phenol) enthielt. Das Gefäß wurde auf Trockeneis gekühlt, um ein Verdampfen der Hydrolyselösung zu vermeiden, und evakuiert (13,3 Pa/100 mTorr). Die Hydrolyse wurde bei 145°C durchgeführt (3 h) (Eckerskorn et al., 1988). Anschließend wurden die Glasfaser-Filter getrocknet und mit 200 $\mu$l Acetonitril-Wasser (50 + 50) eluiert. Ein Aliquot von 150 $\mu$l wurde unter Vakuum getrocknet, mit 50 $\mu$l Wasser extrahiert und erneut getrocknet. Schließlich wurden 50 $\mu$l einer Mischung aus Triethylamin-Wasser-Methanol (1 + 2 + 2) zugesetzt und der Ansatz zur Entfernung überschüssiger HCl nochmals getrocknet. Die Derivatisierung der hydrolysierten Aminosäuren mit o-Phthalaldehyd und die HPLC-Bestimmung der OPA-Aminosäure wurde nach Angaben von Godel et al, 1984 durchgeführt.

N-Terminale Aminosäuresequenz-Analyse (Eckerskorn et al., 1988)

Ca. 10 $\mu$g Protein P 1 wurden auf einem SDS-haltigen 10% Polyacrylamidgel elektrophoretisch aufgetrennt. Die Proteinbande wurden auf eine silikonisierte Glasfaser-Membran (Glossybond, Fa. Biometra, Göttingen) elektrogeblottet und mit Coomassie angefärbt. Anschließend wurde die Proteinbande ausgeschnitten und in die Reaktionskammer des Gasphasen-Proteinsequenators gelegt. Die Membran wurde mit Acetonitril oder Methanol und 20 $\mu$l einer Polybrene Lösung befeuchtet, um das Auswaschen der Probe zu verhindern. Die Durchführung der Bestimmung erfolgt nach der N-terminalen Aminosäuresequenz-Analyse.

HIV-1 RT Test (Tan et al., 1991)

Enzym: Rekombinante HIV-1 Reverse Transkriptase (66 kDa), exprimiert in Escherichia coli (vgl. Tan et al., 1991 und darin zitierte Literatur). Die Aktivität des Enzyms in RT-Testsystemen ist von viralem Enzym nicht zu unterscheiden. Protein-Konzentration: 0,11 mg/ml; Aktivität: Einbau von 238 nM TTP/mg Protein in 10 min bei 37°C.
Testansatz: 50 mM Tris-HCl, pH 8,0, 150 mM KCl, 5 mM $MgCl_2$, 0,5 mM EGTA, 5 mM Dithiothreitol, 0,3 mM Glutathion, 2,5 $\mu$g/ml BSA, 41 $\mu$M poly(A) ($\Sigma_{260}$(mM) = 7,8), 9,5 $\mu$M oligo(dT)$_{12-18}$ ($\Sigma_{265}$(mM) = 5,6), 0,05% Triton X-100, 20 $\mu$m TTP und 0,5 $\mu$Ci ($^3$H)-TTP (Endvolumen 100 $\mu$l).
Probenlösung: 10 $\mu$l, in $H_2O$ oder 25 mM Tris-HCl, pH 8,4.
HIV-1 RT: 10 $\mu$l (0,11 $\mu$g in Testpuffer)

Durchführung:

Die Polymerisationsreaktion wird durch den Zusatz der HIV-1 RT-Lösung zum Testansatz gestartet. Nach einer Inkubationszeit von 1 Stunde (37°C) wird die Reaktion durch Zugabe von 25 $\mu$l einer 0,1 M EGTA-Lösung gestoppt und die Ansätze auf Eis gekühlt.
Ein aliquoter Teil der Ansätze (100 $\mu$l) wird auf DE-81 Filter (Whatman) pipettiert, die nach 15 min bei Raumtemperatur 4 x mit einer wässrigen Natriumhydrogenphosphat-Lösung (5% G/V) und 2 x mit $H_2O$ dest. gewaschen werden. Nach dem Trocknen der Filter wird der Einbau an ($^3$H)-markiertem Thymidintriphosphat im Flüssigszintillationszähler gemessen.
Zur Testung der Enzymhemmung wurden 10 $\mu$l einer Reihenverdünnung der Probe vor der HIV-1 RT-Zugabe zum Testansatz gegeben. Der Thymidineinbau wurde im Verhältnis zu einem Negativ-Kontrollwert (Lösungsmittel) prozentual ausgedrückt (% Hemmung).
Halbmaximale Hemmkonzentrationen ($IC_{50}$) wurden durch Berechnung der linearen Regression aus den Ergebnissen von fünf getesteten Konzentrationen (n = 2) ermittelt. Als Positivkontrolle wurde Fagaronin-Chlorid eingesetzt ($IC_{50}$ = 13 $\mu$M).
Ribosomen-Hemmtest (nach Krawetz et al., 1989)
Zur Messung der Ribosomen-Hemmung wurde ein Retikulozytenlysat-System (nuklease-behandelt, message-abhängig) der Fa. Boehringer Mannheim eingesetzt und optimiert.
Geräte: Öldruck Vakuum Pumpe; Mehrfachfiltergerät für Rundfilterplättchen mit Stahltrichtern, Fa. Hölzel, Dorfen; Liquid Szintillations-System LS 1801, Fa. Beckmann, München; Szintillationscocktail (emulgierend); Biofluor, Fa. NEN Du Pont de Nemours, Bad Homburg

Stammlösungen:

a. Retikulozytenlysat: Lysat mit Zusatz von 125 $\mu$g/ml Kalbsleber tRNA, 40 $\mu$g/ml Hämin, 2 mM EGTA und Kreatin-Kinase (50 U/ml).
b. Translations-Reaktionspuffer: (12,5fach konz.): Je 312,5 $\mu$M von 19 Aminosäuren ohne Leucin, 37,5 mM Fructose-1,6-Bisphosphat, 30 mM cAMP, 12,5 mM ATP, 2,5 mM GTP, 100 mM Kreatinphosphat, 6,25 mM Spermidin, 250 mM HEPES pH 7,6
c. Kaliumacetat: 2,5 M in $H_2O$ dest.
d. Magnesiumacetat: 25 mM in $H_2O$ dest.
e. ($^3$H)-Leucin: L-(4,5-$^3$H)-Leucin, spezifische Aktivität 146 Ci/mMol, Fa. Amersham Buchler GmbH & Co. KG, Braunschweig
f. TMV RNA: 200 $\mu$g/ml Tabak Mosaik Virus RNA (mRNA), Fa. Boehringer Mannheim
g. $H_{20}$ bidest.

Durchführung:

Um Pipettierfehler zu minimieren, wird aus den Reaktionslösungen b - g eine Stammlösung hergestellt, (Standardansatz s.u.) von der jeweils 10 $\mu$l pro Testansatz in Eppendorfgefäßen vorgelegt wird. Nach Zugabe von Wasser, Puffer oder einer Hemmstofflösung wird der Ansatz gut gemischt (Whirli-Mixer) und die Translationsreaktion durch Addition des Retikulozytenlysats gestartet. Nach sorgfältiger Mischung werden die Proben kurz zentrifugiert (1 min, 6000 rpm, Heraeus Biofuge 13) und im Wasserbad bei 30°C für 60 min inkubiert. Dabei ist darauf zu achten, daß die Eppendorfgefäße fest verschlossen sind (2 ml Safe Lock Eppendorfgefäße), um Volumen- und Konzentrationsänderungen zu vermeiden. Anschließend werden die Proben 1:25 mit Wasser verdünnt, ein Aliquot von 25 $\mu$l ($\triangleq$ 1 $\mu$l des Testansatzes) entnommen und die Reaktion mit 500 $\mu$l einer frisch bereiteten 1 M NaOH-Lösung (5% (V/V) $H_2O_2$ enthaltend) gestoppt. Durch Inkubation im Wasserbad bei 37°C (10 min) wird im alkalischen Milieu säurepräzipitierbare Aminoacyl-tRNA deacyliert.

Das synthetisierte Protein wird mit 400 $\mu$l einer frisch hergestellten 50% (G/V) Trichloressigsäure-Lösung (2% (G/V) Caseinhydrolysat enthaltend) ausgefällt und 30 min auf Eis gestellt. Glasfaser-Filter (Whatman GF/C), die maximal 30 min in 5% (G/V) Trichloressigsäure (10 mM Natriumpyrophosphat enthaltend) vorgequollen wurden, werden zum Filtrieren des Gemisches verwendet (Vakuum-Filtergerät). Die Filter werden 3 x mit je 20 ml der zum Vorquellen verwendeten Lösung gewaschen, im Trockenschrank bei 65°C für 10 min getrocknet und anschließend im Flüssig-Szintillationszähler gemessen.

Standardansatz:

```
a. Retikulozytenlysat              10,0 µl (≙ 40% (V/V))

b. Translations-Reaktionspuffer     2,0 µl
   (12,5fach konz.)



c. Kalium-Acetat (2,5 M)          1,0  µl (Endkonzentration 100 mM)

d. Magnesium-Acetat (25 mM)       1,5  µl (Endkonzentration 1,5 mM)

e. (³H)-Leucin                    0,15 µl (≙ 0,75 µCl/Testansatz)

f. TMV RNA (200 µg/ml)            5,0  µl (≙ 1 µg/Testansatz)

g. H₂0 bidest. (Volumenausgleich) 0,35 µl

h. H₂0 oder Hemmstofflösung       5,0  µl (Verdünnung 1:5)


            Gesamtvolumen 25,0 µl
```

Berechnung:

$$\% \text{ Hemmung} = 100 - \frac{\text{Zählrate Testsubstanz (cpm*)}}{\text{Zählrate Negativkontrolle (cpm)}} \times 100$$

* counts per minute

Zellproliferations-Test (Wachinger et al., 1993)

Zellen: nicht infizierte humane T-Lymphomzellen KE37/1; permanent HIV-infizierte T-Lymphomzellen KE37/1-III$_B$; Zellsuspension mit $10^6$ Zellen/ml Medium (von M. Popovic, vgl. Wachinger et al., 1933)

Medium: RPMI 1640, Fa. Gibco, unter Zusatz von Natriumhydrogencarbonat (2 g/l), foetalem Kälberserum (FCS) (10%), und Antibiotica (100 Einheiten Penicillin, 100 $\mu$g Streptomycin und 0,25 $\mu$g Fungizone (Amphotericin B, Fa. Gibco) pro ml)

Kultivierung: 10 $\mu$l der Zellsuspensionen werden in 96 well Mikrotiterplatten (Flachboden) mit 90 $\mu$l Medium gemischt (Tag 0). Das Medium enthält Reihenverdünnungen der Testsubstanzen (8 wells pro Konzentration und Zellinie). Die Zellen werden bei 37°C in 5% $CO_2$ Atmosphäre bebrütet.

MTT-Test (Mosmann, 1983)

MTT-Lösung:

MTT(3-(4,5-Dimethylthiazol-2-yl)-2,5-Diphenyltetrazoliumbromid), 5 mg/ml in PBS, sterilfiltriert

Zur Bestimmung des Zellwachstums werden jedem well 10 $\mu$l der MTT-Lösung zupipettiert. Die Zellkulturen werden 4 h inkubiert (37°C, 5% $CO_2$). Während dieser Zeit wird MTT von stoffwechselaktiven Zellen metabolisiert. Durch Zusatz von 200 $\mu$l 0,04 N HCl in Isopropanol pro well wird die Reaktion gestoppt und der Farbstoff gelöst. Nach einigen Minuten bei Raumtemperatur werden die Platten im Mikroelisa Reader bei 600 nm ausgewertet. Von den gemessenen Werten (OD 600) wird der Medium-Kontrollwert (Kulturmedium mit MTT-Lösung) abgezogen.

COOPER T.G.: Biochemische Arbeitsmethoden, Ed. T.G Cooper, Walter da Gruyter & Co., Berlin - New York (1981)

ECKERSKORN C., MEWES W., GORETZKI H. LOTTSPEICH F.: Eur. J. Biochem. 176, 509-519 (1988)

ECKHARDT A.E., HAYES C.E., GOLDSTEIN I.J.: Anal. Biochem. 73, 192-197 (1976)

ENDO Y., MITSUI K., MOTIZUKI M., TSURUGI K.: J. Biol. Chem. 262, 5908-5912 (1987)

ENDO Y., TSURUGI K., LAMBERT J.M.: Biochem. Biophys. Res. Commun. 150, 1032-1036 (1988)

FAIRBANKS G., STECK T.L., WALLACH D.F.H.: Biochemistry 10, 2606-2617 (1971)

FOA-TOMASI L., CAMPADELLI-FIUME G., BARBIERI L., STIRPE F.: Arch. Virology 71, 323-332 (1982)

FREY M. D. & RADOLA B.J.: Electrophoresis 3, 216-226 (1982)

FURLAN M., PERRET B.A., BECK E.A.: Anal. Biochem. 96, 208-214 (1979)

GERS-BARLAG H.: Dissertation Universität Würzburg (1989)

GODEL H., GRASER T., FÖLDI P., FÜRST P.: J. Chromatogr. 297, 46-61 (1984)

KRAWETZ S.A., NARAYANAN A.S., ANWAR R.A: Can. J. Biochem. Cell Biol. 61, 274-286 (1983)

LAEMMLI U.K.: Nature 227, 680-685 (1970)

LIFSON, et al., WO 88/09123 (1988)

MCGRATH M.S., SANTULLI S., GASTON I.: AIDS Res. Human Retroviruses 6, 1039-1043 (1990)

MCGRATH M.S., HWANG K.M., CALDWELL S.E., GASTON I., LUK K.-C., WU P., NG V.L., CROWE S., DANIELS J., MARSH J., DEINHART T., LEKAS P.V., VENNARI J.C., YEUNG H.W., LIFSON J.D.: Proc. Natl. Acad. Sci. USA 86, 2844-2848 (1989)

MOSMANN T.: J. Immunol. Methods 65, 55-63 (1983)

STIRPE F., BARBIERI L., BATTELLI M.G., FALASCA A.I.. ABBONDANZA A., LORENZONI E., STEVENS W.A.: Biochem. J. 240, 659-665 (1986)

STIRPE F., BARBIERI L., BATELLI M.G., SORIA M. LAPPPI D.A.: Bio/Technology 10, 405-412 (1992)

TAN G.T., PEZZUTO J.M., KINGHORN A.D., HUGHES S.H.: J. Nat. Prod. 54, 143-154 (1991)

TOMLINSON J.A., WALKER V.M., FLEWETT T.H., BARCLAY G.R.: J. Gen. Virol. 22, 225-232 (1974)

TSAO S.W., YAN K.T., YEUNG H.W.: Toxicon 24, 831-840 (1986)

WACHINGER M., SAMTLEBEN R., GERHÄUSER C., WAGNER H., ERFLE V.: Res. Exp. Med. 193, 1-12 (1993)

YEUNG H.W., LI W.W., FENG Z., BARBIERI L., STIRPE F.: Int. J. Peptide Protein Res. 31, 265-268 (1988)

**Patentansprüche**

1. Ribosomen-inaktivierendes Protein mit einer Molmasse von 31 kDa ± 0,4 kDa, bestimmt in der SDS-PAGE, einem IEP von 8 bis 9 und der folgenden N-terminalen Aminosäuresequenz.

```
1
Asp-    Ile-  Ala-  Leu-  Asp-  Leu-  Ser-  Thr-  Ala-  10
                                                        Thr-
11                                                      20
(Lys)-  Ala-  Ser-  Tyr-  Ser-  Ala-  Phe-  Ile-  Thr- His
21            23
(Trp)-  Arg-  Asn
```

2. Verfahren zur Isolierung eines Ribosomen-inaktivierenden Proteins nach Anspruch 1, bei dem man Pflanzenteile, insbesondere Samen von Cucurbita-Arten durch Mahlen zerkleinert, mit lipophilen organischen Lösungsmitteln extrahiert, den getrockneten Rückstand mit einer salzhaltigen Pufferlösung extrahiert, die Proteine aus dem Extrakt anreichert, dann die basischen Proteinfraktion durch Anionenaustausch-Chromatographie weiter anreichert und die im Ribosomen-Hemmtest aktiven Fraktionen durch Gelfiltration zur Isolierung des erfindungsgemäßen Proteins weiter aufreinigt.

3. Verfahren nach Anspruch 2, bei dem man als lipophiles Extraktionsmittel Aceton, n-Hexan, Petrolether, Dichlormethan, Ethanol oder Mischungen davon verwendet.

4. Verfahren nach einem der Ansprüche 2 und 3, bei dem man als salzhaltigen Puffer PBS verwendet.

5. Verfahren nach einem der Ansprüche 2, 3 oder 4, bei dem man die Anreicherung der Proteine durch fraktionierte Ammoniumsulfatfällung zu 30% und 90% durchführt.

6. Verfahren nach Anspruch 2, 3 oder 4, bei dem man die Anreicherung der Proteine durch Fällung mit einem Niedrigalkylalkohol oder Aceton durchführt.

7. Verfahren nach einem der Ansprüche 1 bis 6, bei dem man die Ionenaustausch-Chromatographie zunächst als Anionenaustausch-Chromatographie durchführt, dann die nichtgebundene Fraktion durch Kationenaustausch-Chromatographie fraktioniert.

8. Verfahren nach Anspruch 7, bei dem man als Kationenaustauschmaterial Carboxymethyl-Cellulose verwendet.

9. Verfahren nach Anspruch 8, bei dem man die gebundene Proteinfraktion mit einem linearen NaCl-Gradient von 0 bis 0,5 M in 10 mM Natriumphosphatpuffer pH 6 eluiert.

10. Verfahren nach einem der Ansprüche 1 bis 9, bei dem man die im Ribosomen-Hemmtest aktiven Fraktionen durch Gelfiltrations-Chromatographie über Sephadex G 75 trennt, und gegebenenfalls diesen Schritt wiederholt.

11. Verfahren nach Anspruch 2, bei dem man gegebenenfalls zusätzlich zu den genannten Schritten eine affinitätschromatographische Trennung an geeigneter Stelle durchführt.

12. Verfahren nach einem der Ansprüche 2 bis 11, bei dem man die Einzelschritte in jeder geeigneten Reihenfolge durchführt.

13. Verfahren nach Anspruch 2, bei dem man zur Proteinanreicherung präparative elektrophoretische Methoden einsetzt.

14. Verfahren nach Anspruch 2, bei dem man das Protein nach Anspruch 1 aus in Zellkultur kultivierten Pflanzenteilen isoliert.

15. Verfahren nach Anspruch 2, bei dem man das Protein nach Anspruch 1 durch gentechnologische Verfahren erzeugt.

16. Pharmazeutische Zubereitung enthaltend ein Protein nach Anspruch 1 in Verbindung mit bekannten und üblichen Träger- und Verdünnungsstoffen.

17. Pharmazeutische Zubereitung nach Anspruch 16 zur Behandlung von Viruserkrankungen, vorzugsweise von durch Retroviren, insbesondere durch HIV verursachte Erkrankungen.

18. Pharmazeutische Zubereitung nach Anspruch 16 zur Behandlung von proliferativen Tumoren, vorzugsweise zur Behandlung der Hyperplasie, insbesondere zur Behandlung von Prostata-Hyperplasie.

ABB. 1

ISOLIERUNGSSCHEMA ZUR DARSTELLUNG DES ERFINDUNGSGEMÄßEN PROTEINS (P1)

**Gemahlene Kürbiskerne**

Extraktion mit lipophilen organischen Lösungsmitteln (Raumtemperatur)

getrocknetes Samenmehl

Extraktion mit NaCl-haltigen Puffer

Filtration

**Rohextrakt**

Ammoniumsulfat (AS) Fällung
30% Sättigung
90% Sättigung

Zentrifugation
Dialyse des 90% AS-Präzipitats
gegen Puffer

**Proteinangereicherter Extrakt**

Anionenaustausch-Chromatographie

**Neutralfraktion**                    saure Proteine

Pufferwechsel

Kationenaustausch-Chromatographie

a

Salz-Gradient

**b**    Test (Ribosomen-Hemmtest)

Gelfiltration

bA        **bB**        bC        bD

Rechromatographie
(Gelfiltration)

**P 1**

21

Abb. 2

## Hemmung der HIV-1 RT durch Protein P 1

**% Kontrollaktivität** (y-Achse, 0 bis 100)

Protein P1 [µg/ml] (x-Achse, 1 bis 100)

-□- Test 1
-△- Test 2

Abb. 3  Stoffwechselaktivität HIV-infizierter und nicht infizierter T-Lymphom-Zellkulturen in Gegenwart verschiedener Konzentrationen von Protein P1

**Stoffwechselaktivität (OD 600)** (y-Achse, 0 bis 1,2)

Protein P1 [µg/ml] (x-Achse, 0 bis 10)

-△- KE37/1 (2 Tage)
-▽- KE37/1 (4 Tage)
-•- KE37/1-IIIB (2 Tage)
-Ɛ- KE37/1-IIIB (4 Tage)

Abb. 4  Darstellung der Dosisabhängigkeit der Wachstumshemmung HIV-infizierter und nicht infizierter T-Lymphomzellen durch Protein P1

nach einer Inkubationszeit von 2 Tagen

Abb. 5  nach einer Inkubationszeit von 4 Tagen